# EUROPÄISCHE PATENTSCHRIFT

(11) **EP 1 149 849 B1**
(45) Veröffentlichungstag und Bekanntmachung des Hinweises auf die Patenterteilung: **28.05.2003**
(21) Anmeldenummer: 01107566.0
(22) Anmeldetag: 27.03.2001
(51) Int. Cl.: C08G 18/00, C08J 9/00, C12N 11/08

(54) **Verfahren zur Herstellung von kovalent gebundenen biologisch aktiven Stoffen an Polyurethanschaumstoffen sowie Verwendung der geträgerten Polyurethanschaumstoffe für chirale Synthesen**
Process for the production of covalently bound biologically active materials on polyurethane foams and the use of such carriers for chiral syntheses
Procédé pour la préparation de materieux bioactifs liées de maniere covalente à des mousses de polyuréthanne rt l'utilisation de supports de mousses polyuréthanne pour les sythèses chirales

(30) Priorität: 19.04.2000 DE 10019380
(43) Veröffentlichungstag der Anmeldung: 31.10.2001
(73) Patentinhaber: BASF AKTIENGESELLSCHAFT, 67056 Ludwigshafen (DE)
(72) Erfinder: Falke, Peter, Dr., 01987 Schwarzheide (DE); Hendreich, Regina, 01945 Frauendorf (DE); Stürmer, Rainer, Dr., 67127 Rödersheim-Gronau (DE); Friedrich, Thomas, Dr., 64283 Darmstadt (DE)

(56) Entgegenhaltungen:
- WO-A-00/66092
- DE-A- 2 413 137
- DE-A- 4 131 546
- US-A- 3 929 574

## Beschreibung

Die Erfindung betrifft ein Verfahren zur Herstellung von kovalent gebundenen biologisch aktiven Stoffen an Polyurethanschaumstoffen sowie den Einsatz der geträgerten Polyurethanschaumstoffe für chirale Synthesen.

Die Herstellung von Polyurethanschaumstoffen durch Umsetzung von Polyisocyanaten mit Verbindungen mit mindestens zwei reaktiven Wasserstoffatomen sowie weiteren Hilfs- und Zusatzstoffen ist seit langem bekannt und wurde vielfach beschrieben. Hauptsächlich werden als Polyurethanschaumstoffe Hart- und Weichschäume verwendet. Je nach Einsatzzweck werden dabei offenzellige oder geschlossenzellige Schäume bevorzugt.

Eine zusammenfassende Übersicht über die Herstellung von Polyurethanschaumstoffen wird z.B. im Kunststoff-Handbuch, Band VII, "Polyurethane", 1. Auflage 1966, herausgegeben von Dr. R. Vieweg und Dr. A. Höchtlen, sowie 2. Auflage, 1983, und 3. Auflage, 1993, jeweils herausgegeben von Dr. G. Oertel (Carl Hanser Verlag, München), gegeben.

Enzyme werden aufgrund ihrer Selektivität und hohen katalytischen Aktivität in steigendem Ausmaß in der Lebensmittel-, Pharma- und chemischen Industrie eingesetzt. Technologische Nachteile bei der Verwendung von Enzymen bestehen insbesondere darin, dass diese häufig wegen ihrer Substratlöslichkeit aus dem Reaktionsmedium nicht befriedigend abgetrennt werden können. Neben dem damit einhergehenden Verlust an diesen teuren Biokatalysatoren entstehen zusätzliche Aufwendungen durch entsprechend erforderliche Reiniungsprozesse.

Durch eine geeignete Immobilisierung der wasserlöslichen Enzyme können eine Reihe von vorteilhaften Effekten erreicht werden:
- leichte Abtrennbarkeit des Biokatalysators (Enzym) aus der Reaktionslösung;
- Wiederverwertbarkeit des geträgerten Enzyms (Senkung der spezifischen Katalysatorkosten);
- keine Nachbehandlung (Deaktivierung, Reinigung) der Prozesslösung erforderlich;
- kontinuierliche Prozessführung möglich (bessere Prozesskontrolle)

In EP-A-0562371 und EP-A-0562373 wird die Immobilisierung biochemischer Substanzen, insbesondere von Enzymen, beschrieben. Im Vergleich zur physikalischen Adsorption von Enzymen, bei der eine leichte Desorption dieser Enzyme beobachtet wird, erfolgt bei einer kovalenten Enzymfixierung eine wesentlich beständigere Bindungsknüpfung zwischen Substratoberfläche und Enzymmolekül. Diese Bindungsknüpfung wird nach diesen EP dadurch realisiert, dass ein ungesättigtes epoxyfunktionelles Polysiloxan auf ein Trägermaterial aufgebracht und vernetzt wird. Das Enzym wird danach kovalent über die so eingebrachten Epoxidgruppen gebunden.

Nach DE-A-4131546 wird Lipase an einem kommerziell erhältlichen Träger (Eupergit C - Röhm Pharma Deutschland) über vorhandene Epoxidgruppen kovalent verknüpft. Mit Hilfe des so immobilisierten Enzyms sollen chirale Alkohole erzeugt werden.

In US-A-4342834 wird ein Enzym bei Beibehaltung oder Steigerung seiner katalytischen Aktivität an ein Polyurethan gebunden. Dabei wird vorzugsweise das Enzym aus wässriger Lösung mit dem Polyurethan in Kontakt gebracht, bevor das Polyurethan verschäumt wird. Beim Verschäumen wird dann darauf geachtet, dass durch die Reaktionswärme keine Denaturierung des Enzyms eintritt.

In EP-A-0089165 werden Versuche japanischer Autoren zitiert, Zellen einer Zellsuspension mit Hilfe eines kommerziell erhältlichen Prepolymers (HYPOL, Fa. Grace) unter Ausbildung eines Schaumes, der diese immobilisierten Zellen enthält, zur Reaktion zu bringen. Diese so erhaltenen Schäume weisen insbesondere eine mangelhafte mechanische Beständigkeit auf, was zu technologischen Problemen führt. Als Lösung wird die Immobilisierung an einem vernetzbaren Polyazetidinprepolymeren genannt.

Nach EP-A-0859051 werden Enzyme an wasserunlösliche Polymere, die quaternäre Aminogruppen tragen, fixiert.

In WO-A-9746590 und -9746267 werden verschiedene Methoden zur Immobilisierung von bioaktiven Materialien genannt. Auf einem Träger wird zunächst eine Schicht eines polymeren oberflächenaktiven Materials aufgebracht. Dieses aufgebrachte Material wird mit-Hilfe eines Vernetzers in-situ vernetzt. Als Vernetzer werden u.a. Vinylverbindungen, Imidazole, Epoxide, Isocyanate, Anhydride usw. genannt. Auf diese Schicht wird letztlich eine zweite Schicht eines hydrophilen Polymers aufgebracht und so mit der Basisschicht verbunden. Das bioaktive Molekül wird über die zweite Schicht angeknüpft.

In DE-A-19653669 werden Trägermatrices mit reaktiven Gruppen (Aktivierung u.a. durch Glutaraldehyd, Bromcyan, Imidazolderivate) genannt, mit denen Immunoglobuline aus biologischen Flüssigkeiten abgetrennt werden können.

Nach CA-A-1264898 wird versucht, unter Verwendung von Gelatine und aliphatischen Diisocyanaten eine Enzymbindung an hydrophilen Gelen zu erzeugen.

Wang und Ruckensten (Biotechnol. Progr. 1993, 9, 661-65) verwandten poröse Polyurethan-Partikel, um Lipase zunächst zu adsorbieren. Durch eine Umsetzung mit Glutaraldehyd wurde versucht, durch die erzeugte Vernetzung eine Stabilisierung der Immobilisate zu erreichen.

O'Reilly und Crawford (Appl. and Environmental Microbiology, 1989, 2113-18) versuchten mit Hilfe eines Polyurethan-Prepolymers (HYPOL) eine Zellsuspension zu immobilisieren, wobei in diesem Falle die Suspension lediglich mit dem Prepolymeren verrührt und zur Reaktion gebracht wird.

In US-A-3672955 werden wasserlösliche Enzyme zur Fixierung mit einem Isocyanat in wässriger Lösung verrührt, wobei noch poröse Materialien zusätzlich mit verwendet werden können. Über die so erzeugten Harnstoffe sollen die Enzyme kovalent gebunden sein.

Nach SU 1472503 wird ein Enzym zunächst an einen silikatischen Träger (Silochrom) adsorbiert. Das so hergestellte Material wird dann nachfolgend in ein Polyurethan eingeschäumt.

In EP-A-0571748 wird die Tatsache diskutiert, dass Matrices aus Polyurethan-Hydrogelen zwar gute mechanische Eigenschaften aufweisen, die vorhandenen freien Isocyanatgruppen jedoch eine hohe Toxizität gegenüber biologischen Materialien besitzen. Dadurch ist eine beträchtliche Eigenschaftsschädigung zu erwarten. Deshalb wird vorgeschlagen, zunächst eine Verkappung der Isocyanatgruppen durch ein Bisulfitaddukt zu erzeugen. Zu dieser wässrigen Lösung dieses Bisulfitadduktes wird die Zellmasse gegeben. Dieses Gemisch wird in eine Na-Alginatlösung eingetropft. Die sich ausbildende Alginathülle wirkt stabilisierend und kann nachfolgend in einem Phosphatpuffer abgelöst werden.

Burg et al. (Angew. Makromolekulare Chemie, 157, (1988), 105-21) beschreiben die Herstellung spezieller synthetischer Polymere 5 durch Suspensionspolymerisation von Vinylacetat und N,N'-Divinylethylenharnstoff. Nach entsprechender Aufarbeitung werden durch Umsetzung mit Epichlorhydrin reaktive Epoxidgruppen an der Polymeroberfläche zur Enzymbindung erzeugt.

Die dem Stand der Technik entsprechenden Vorschläge gestatten durchaus in Einzelfällen eine gewisse Immobilisierung biologisch aktiver Spezies, die jedoch häufig mit einem Abfall der katalytischen Aktivität verbunden ist. Es besteht noch ein hinreichendes Verbesserungspotential. Insbesondere ist es erforderlich, Lösungen zu entwickeln, die es gestatten, sowohl in wasserfreien Medien als auch aus wässrigen Lösungen und/oder Suspensionen biologisch aktive Spezies insbesondere über Isocyanatgruppen an Polyurethanoberflächen dauerhaft unter Beibehalt der katalytischen Aktivität zu binden. Dabei muss gewährleistet sein, dass die hohe Toxizität der Isocyanatgruppen zu keiner Schädigung der biologisch aktiven Spezies führt.

Aufgabe der Erfindung war es demzufolge, ein Verfahren zu entwickeln, das eine dauerhafte Fixierung von biologisch aktiven Stoffen, insbesondere Enzymen, an Polyurethanschäumen ermöglicht, wobei eine ausreichende mechanische Festigkeit des Trägermaterials gewährleistet sein sollte. Der so hergestellte Biokatalysator-Polyurethanträger-Verbund sollte bei hoher Selektivität in chiralen Synthesen, insbesondere bei Umesterungsverfahren, einsetzbar sein.

Diese Aufgabe konnte überraschenderweise dadurch gelöst werden, dass Polyurethanschäume entwickelt wurden, die reaktive Gruppen an der Schaumstoffoberfläche aufweisen und somit als Trägermaterial für eine dauerhafte Fixierung von biologisch aktiven Stoffen geeignet sind.

Gegenstand der Erfindung sind demzufolge ein Verfahren zur Herstellung von kovalent gebundenen biologisch aktiven Stoffen an Polyurethanschaumstoffen sowie solche mit biologisch aktiven Materialien geträgerten Polyurethanschaumstoffe selbst.

Gegenstand der Erfindung ist weiterhin deren Verwendung für chirale Synthesen.

Wir fanden bei unseren Untersuchungen überraschenderweise, dass bei Einsatz von Polyurethanschaumstoffen, die reaktive Gruppen an der Schaumstoffoberfläche enthalten, biologisch aktive Materialien dauerhaft auf der Schaumstoffoberfläche verankert und damit immobilisiert werden können, ohne dass diese ihre Aktivität verlieren. Das erfindungsgemäße Ergebnis war deshalb überraschend, da bei einer zu starken Ausprägung einer kovalenten Bindung mit der Gefahr zu rechnen ist, dass das katalytisch aktive Zentrum der Enzyme geschädigt wird. In der Folge sinkt die Aktivität. Bei einer zu schwachen kovalenten Bindung kommt es zu einer Desaktivierung infolge Ablösung des Enzyms vom Träger.

Besonders vorteilhaft und überraschend ist, dass das Aufbringen der biologisch aktiven Materialien auf den Träger im wässrigen Medium erfolgen kann. Dies erspart erhebliche Aufwendungen. Zu erwarten wäre gewesen, dass die auf der Schaumstoffoberfläche vorhandenen reaktiven NCO-Gruppen durch das Wasser deaktiviert würden.

Die für den erfindungsgemäßen Zweck verwendeten Polyurethanschaumstoffe werden auf übliche Art und Weise durch Umsetzung von organischen und/oder modifizierten organischen Polyisocyanaten (a) mit gegenüber Isocyanaten reaktive Wasserstoffatome aufweisenden Verbindungen (b) in Gegenwart von Hilfs- und Zusatzstoffen (c), hergestellt. Die hierfür einsetzbaren Ausgangsstoffe und Verfahrensbedingungen werden weiter unter näher beschrieben.

Erfindungsgemäß werden vorzugsweise Polyurethanhartschaum- oder -halbhartschaumstoffe verwendet, da diese Materialien eine für den vorgesehenen Einsatz ausreichende mechanische Festigkeit aufweisen. Es ist aber durchaus auch möglich, weichere Schaumstoffe einzusetzen.

Die erfindungsgemäß erforderlichen reaktiven Gruppen an der Schaumstoffoberfläche sind insbesondere NCO-Gruppen. Neben den NCO-Gruppen können als weitere reaktive Gruppen beispielsweise Epoxidgruppen, Säuregruppen und/oder phenolische OH-Gruppen vorhanden sein.

Um die erfindungsgemäß erforderlichen reaktiven Gruppen an der Schaumstoffoberfläche zu erzeugen, sozusagen im Schaumgerüst zu verankern, werden unterschiedliche Verfahren angewandt. Das entsprechende Ergebnis kann man beispielsweise durch Zugabe von, gegebenenfalls zusätzlichen, Mengen an geeigneten Ausgangsstoffen oder durch eine nachträgliche Tränkung des Polyurethanschaumstoffes mit solchen Stoffen erzielen.

Durch Steuerung des Reaktionsumsatzes der Polyurethanreaktion können reaktive NCO-Gruppen an den Schaumstoffoberflächen erzeugt werden. Dazu wird der Anteil an Isocyanatgruppen über das Verhältnis von Isocyanatgruppen zu Hydroxylgruppen bei der Verschäumung, zumeist als Kennzahl bezeichnet, beeinflußt. Es hat sich dabei als vorteilhaft erwiesen, wenn bei der Verschäumung der Polyurethane eine Kennzahl >100, insbesondere >130, verwendet wird, um eine hinreichende Zahl reaktiver NCO-Gruppen an der Oberfläche des Schaumes zu erzeugen. Da während der Schäumreaktion insbesondere die besonders reaktiven NCO-Gruppen, die auch die nachgewiesene toxische Wirkung auf die biologisch aktiven Stoffe ausüben, zur Reaktion gebracht werden können, zeigte es sich überraschenderweise, dass die so verbleibenden Isocyanatgruppen über eine abgestufte Reaktivität verfügen, die die Bindung der biologisch aktiven Verbindungen an der Schaumstoffoberfläche bewerkstelligt, ohne eine dauerhafte Schädigung hervorzurufen. Aufgrund der so abgeminderten Reaktivität, die auch durch die Glastemperatur des Polymeren bestimmt wird, stehen diese reaktiven Gruppen über einen längeren Zeitraum zur Verfügung. Ihre Selektivität ist auch in wässriger Umgebung hinreichend groß für die Bindung der biologisch aktiven Spezies.

Vorteilhafterweise sollte die Weiterverarbeitung des mit reaktiven NCO-Gruppen auf der Schaumstoffoberfläche versehenen Polyurethanträgers zu dem erfindungsgemäßen Enzym-Träger-Verbund unmittelbar nach seiner Herstellung erfolgen. Ist eine längere Lagerung erforderlich, so sollte sie unter Feuchtigkeitsausschluss vorgenommen werden, um eine Reaktion der NCO-Gruppen mit dem Wasser und somit eine Deaktivierung des Polyurethanträgers zu verhindern.

Vorteilhaft ist es auch, durch eine nachträgliche Tränkung des Polyurethanschaumstoffes mit Di- und/oder Polyisocyanaten und nachfolgende Konditionierung zusätzliche reaktive NCO-Gruppen an der Schaumstoffoberfläche zu erzeugen. Dabei können auch Isocyanate mit unterschiedlich reaktiven NCO-Gruppen Verwendung finden. Hierfür eignen sich insbesondere folgende Isocyanate: Diphenylmethandiisocyanate, Toluylendiisocyanate und Polyphenylenpolymethylenpolyisocynat, jeweils allein oder in beliebigen Gemischen untereinander.

Für den Tränkvorgang wird der vorzugsweise offenzellige Schaumstoff - besonders bevorzugt nach einem vorherigen Zerkleinerungsvorgang - mit einer Lösung eines Isocyanates in einem organischen Lösungsmittel behandelt. Nach dem Entfernen des Lösungsmittels steht dann ein Polyurethan mit zusätzlichen Isocyanatgruppierungen an der zugänglichen Oberfläche für die kovalente Bindungsknüpfung mit biologisch aktiven Stoffen, wie beispielsweise Enzymen, zur Verfügung.

Es hat sich gezeigt, dass ein kurzzeitiges Temperieren des Schaumstoffes über ca. 1 Stunde bei 80°C mit einer nachfolgenden Aufbringung eines reaktiven Diphenylmethandiisocyanates einen positiven Einfluß auf das Vorhandensein der reaktiven zugänglichen NCO-Gruppen auf dem kornförmigen Polyurethanmaterial besitzt. Nach dem Tränkprozess mit diesem Isocyanat wird das überschüssige Lösungsmittel entfernt. Unmittelbar danach kann dann die überstehende Fermenterlösung, die die biologisch aktiven Stoffe enthält, mit dem so aktivierten Trägermaterial umgesetzt werden.

Neben den Isocyanatgruppierungen können weitere reaktive Gruppen, wie beispielsweise Epoxidgruppen, Säuregruppen und/oder phenolische OH-Gruppen, vorhanden sein.

In einigen Fällen ist es von Vorteil, wenn zusätzliche funktionelle Gruppen auf der Schaumstoffoberfläche anwesend sind, da dadurch die Bindung der biologisch aktiven Materialien an der Schaumstoffoberfläche verbessert werden kann.

Durch Verwendung von OH-funktionellen Epoxiden können reaktive Epoxidgruppen an der Schaumstoffoberfläche erzeugt werden. Als OH-funktionelle Epoxide kommen hierfür beispielsweise infrage: Glycidol und 1-Hydroxybutylenoxid, vorzugsweise eingesetzt wird Glycidol.

Durch Verwendung von säuregruppentragenden Verbindungen können reaktive Säuregruppen an der Schaumstoffoberfläche erzeugt werden. Als säuregruppentragende Verbindungen kommen hierfür vorzugsweise infrage: OH-funktionelle Säuren, wie beispielsweise Glycidolsäure, Hydroxybuttersäure, Dimethylolpropionsäure und/oder säuregruppenhaltige Prepolymere, wie beispielsweise NCO-Prepolymere auf Basis von Diphenylmethandiisocyanat, Polyolbestandteilen und Dimethylolpropionsäure. Besonders bevorzugt eingesetzt werden Glycidolsäure und Dimethylolpropionsäure.

Durch Verwendung von Phenolen, beispielsweise Mitverschäumen entsprechender Ausgangsstoffe oder nachträgliches Trägern, können phenolische OH-Gruppen an der Schaumstoffoberfläche erzeugt werden. Als phenolische Verbindungen kommen hierfür vorzugsweise infrage: Calixarenderivate, wie z.B. C₉- oder C₁₀-Calixaren. Besonders bevorzugt eingesetzt wird C₉-Calixaren.

Es ist besonders vorteilhaft, Calixarene in die Polyurethanmatrix einzubauen. Sie sind von hoher Beständigkeit und haben die Fähigkeit, gegebenenfalls als Coenzyme geeignete Metallionen zu fixieren.

Als Calixarene finden Verbindungen Verwendung, wie sie u.a. in Angewandte Chemie 107 (1995) 785-818 durch V. Böhmer beschrieben sind. Ihre Synthese basiert im Wesentlichen auf der Umsetzung von verschiedenen Phenolen oder Resorcinderivaten und Formaldehyd.

Neben den genannten, vorzugsweise zur Erreichung des erfindungsgemäßen Ergebnisses eingesetzten Verbindungen, können auch weitere reaktive Gruppen, wie beispielsweise Aminogruppen, auf der Schaumstoffoberfläche vorhanden sein. Aminogruppen können beispielsweise erzeugt werden, indem freie Isocyanatgruppen an der Oberfläche des Schaumes mit Polyaminen abgesättigt werden.

Die Menge der auf den Polyurethanträger aufzubringenden reaktiven Gruppen hängt vom jeweiligen Enzymtyp und den Applikationsbedingungen ab und kann durch geeignete einfache Tests eingestellt werden.

Das Vorhandensein der reaktiven Gruppen auf der Schaumstoffoberfläche lässt sich durch spektroskopische Verfahren, insbesondere die IR-Spektroskopie, kontrollieren. Dabei werden jedoch auch funktionelle Gruppen angezeigt, die den biologisch aktiven Stoffen gegebenenfalls nicht zugänglich sind. Sehr gut geeignet ist ein Vergleichsexperiment, das unter ansonsten identischen Bedingungen mit und ohne Anwesenheit der reaktiven Gruppen durchgeführt wird. Bei Nichtanwesenheit derartiger zugänglicher Gruppen kommt es in der Regel zu einem schnellen Aktivitätsabfall der Enzyme.

Zur Durchführung des erfindungsgemäßen Verfahrens ist es vorteilhaft, eine polyolische Komponente, die die gegenüber Isocyanaten reaktive Wasserstoffatome aufweisenden Verbindungen (b) und zumeist auch die zur Herstellung der Polyurethanschaumstoffe notwendigen Katalysatoren, Treibmittel sowie sonstigen Hilfs- und Zusatzstoffe (c) enthält und üblicherweise als Polyolkomponente bezeichnet wird, mit der Isocyanatkomponente zu einem Polyurethanschaumstoff umzusetzen. Vorteilhafterweise werden die Verbindungen mit den einzubringenden reaktiven Gruppen vor dem Verschäumen der Polyolkomponente zugesetzt. Es ist jedoch prinzipiell auch möglich, ein Material mit zusätzlichen reaktiven Gruppen der Isocyanatkomponente zuzusetzen.

Es ist auch möglich, nach entsprechender Konfektionierung des Polyurethanträgers eine nachträgliche Aufbringung der genannten reaktiven Gruppierungen, beispielsweise durch einen Tränkvorgang mit entsprechender Konditionierung, zu realisieren.

Zweckmäßigerweise ist der Schaumstoff vorher auf die gewünschte Korngröße zu zerkleinern oder der Porendurchmesser ist hinreichend groß zu erzeugen, damit eine Tränklösung auch alle Teile der Festkörperoberfläche erreichen kann.

Die erfindungsgemäßen Polyurethanschaumstoffe sollen eine möglichst große Oberfläche für den Kontakt mit den biologisch aktiven Materialien, die auf der Schaumstoffoberfläche zu fixieren sind, aufweisen.

Die Zugänglichkeit der reaktiven Stellen kann durch eine Einstellung der Offenzelligkeit der Schaumstoffe oder durch eine Zerkleinerung der Polyurethanschaumstoffe, z.B. über einen Mahlprozess, erzeugt werden.

Die Einstellung der Offenzelligkeit der Schaumstoffe erfolgt beispielsweise durch geeignete zellöffnende Materialien und Verwendung handelsüblicher Silikonstabilisatoren. Bewährt hat sich eine Offenzelligkeit von >90 %.

Werden zerkleinerte Schaumstoffe eingesetzt, verwendet man vorteilhafterweise Korngrößen < 5 mm, wobei bei zu kleinen Korngrößen ein erhöhter Strömungswiderstand in den Reaktionsgefäßen zu erwarten ist.

Weiterhin kann es vorteilhaft sein, die Polyurethanschaumstoffe hydrophil einzustellen, um eine optimale Benetzung des Schaumstoffs mit dem Reaktionsmedium zu gewährleisten. Die Hydrophilie der Polyurethanschaumstoffe kann beispielsweise durch die Verwendung von Polyetherolen mit einem hohen Gehalt an Ethylenoxid in der Kette erhöht werden.

Es ist auch möglich, die erfindungsgemäßen Schaumstoffe hydrophob einzustellen. Derartige hydrophobe Schaumstoffe erhält man beispielsweise durch eine bevorzugte Verwendung von Polyolen mit einem erhöhten Gehalt an Propylenoxid in der Polyetherkette. Diese Schaumstoffe haben in der Regel eine gute Gerüststabilität.

Die beschriebenen erfindungsgemäßen Polyurethanschaumstoffe mit reaktiven Gruppen an der Schaumstoffoberfläche eignen sich insbesondere zur kovalenten Bindung von biologisch aktiven Materialien.

Unter biologisch aktivem Material werden Produkte verstanden, die biologische Prozesse zu aktivieren vermögen. Hierzu gehören und sind erfindungsgemäß einsetzbar beispielsweise Zellkulturen, Zellsuspensionen, Mikroorganismen und insbesondere Enzyme. Sie können für den Anwendungsfall dieser Erfindung sowohl als festes Material oder als Lösung oder gegebenenfalls als Suspension in einem wäßrigen und/oder organischen Medium vorliegen. Enzyme werden in reiner Form oder gegebenenfalls als überstehende Fermenterlösung aus der Fermentation von Zellverbänden zur Verfügung gestellt. Insbesondere Enzyme sind zu chiralen Synthesen befähigt. Eine häufig angewandte Synthese ist dabei die Umesterung geeigneter Rohstoffe bei möglichst selektiver Ausbeute an den gewünschten chiralen Estern. Als Enzym wird dabei vorzugsweise eine Lipase angewandt, die durch Fermentation von Pseudomonas Plantarii gewonnen wird. Nach einer entsprechenden Gefriertrocknung steht diese Lipase als festes Pulver zur Verfügung, wobei dieser Aufbereitungsschritt kostenintensiv ist. Von besonderem Interesse ist jedoch eine Fermenterlösung, die nach dem Abzentrifugieren des Zellmaterials verbleibt und die das zu fixierende Enzym in einer wäßrigen Suspension enthält.

Die Herstellung der mit biologisch aktiven Materialien geträgerten Polyurethanschaumstoffe erfolgt durch Inkontaktbringen des auf der Oberfläche reaktive Gruppen enthaltenen Polyurethanträgers mit biologisch aktiven Materialien, vorteilhafterweise durch Verrühren des Trägermaterials mit beispielsweise einer wässrigen Fermenterbrühe. Als Andockstelle dienen vorzugsweise vorhandene reaktive Aminogruppen des biologisch aktiven Materials. Diese können mit den reaktiven Oberflächengruppierungen des Trägers reagieren.

Der Enzym-Träger-Verbund wird anschließend abgetrennt und getrocknet. Er weist vorteilhafterweise eine Quellung der geträgerten biologisch aktiven Materialien von weniger als 10% auf.

Die erfindungsgemäßen mit biologisch aktiven Materialien geträgerten Polyurethanschaumstoffe werden insbesondere für chirale Synthesen, insbesondere für selektive Umesterungsreaktionen eingesetzt. Ganz besonders bevorzugt werden dazu Lipase-Polyurethan-Materialien. Bei dieser enzymatischen Veresterungsreaktion können sehr milde Reaktionsbedingungen eingestellt werden, was die Verwendung von wärmeempfindlichen Materialien gestattet.

Zu den für die Durchführung des erfindungsgemäßen Verfahrens notwendigen weiteren Einsatzprodukten sowie Hilfs- und/oder Zusatzstoffen ist im Einzelnen Folgendes zu sagen:

Die Herstellung der als Trägermaterial dienenden Polyurethanschaumstoffe erfolgt üblicherweise durch Umsetzung von organischen und/oder modifizierten organischen Polyisocyanaten (a) mit gegenüber Isocyanaten reaktive Wasserstoffatome aufweisenden Verbindungen (b) in Gegenwart von Hilfs- und Zusatzstoffen (c).

Als organische und/oder modifizierte organische Polyisocyanate (a) können die üblichen und bekannten (cyclo)aliphatischen und insbesondere aromatischen Polyisocyanate eingesetzt werden. Beispiele für (cyclo)aliphatische Polyisocyanate sind Hexamethylendiisocyanat-1,6 und Isophorondiisocyanat, Beispiele für aromatische Polyisocyanate sind 2,4- und 2,6-Toluylendiisocyanat (TDI), 4,4'-, 2,4'- und 2,2'-Diphenylmethandiisocyanat (MDI), Polyphenylen-polymethylen-polyisocyanate (Roh-MDI), 1,5-Naphthylendiisocyanat. Die genannten Isocyanate können auch modifiziert sein, beispielsweise durch Einbau von Carbodiimidgruppen. Häufig werden die Polyisocyanate auch in Form von Prepolymeren eingesetzt. Dabei handelt es sich um Umsetzungsprodukte der genannten Polyisocyanate mit Polyolkomponenten. Zumeist werden sogenannte Isocyanatprepolymere verwendet, das heißt solche Umsetzungsprodukte von Polyolen und Polyisocyanaten, die am Kettenende freie Isocyanatgruppen aufweisen. Die Prepolymere und Quasiprepolymere und ihre Herstellung sind allgemein bekannt und vielfach beschrieben. Für das erfindungsgemäße Verfahren werden insbesondere Prepolymere mit einem NCO-Gehalt im Bereich von 25 bis 3,5 Gew.-% eingesetzt. Als Polyole zur Herstellung der Prepolymere und Quasiprepolymere werden vorzugsweise Polyetherole, insbesondere 2- und 3-funktionelle Ethylenoxid- und/oder Propylenoxid-Anlagerungsprodukte mit Molekulargewichten von 1000 bis 8500, Polytetramethylenglykole mit Molekulargewichten von 500 bis 3000, Polyesterole mit Molekulargewichten im Bereich von 500 bis 5000 und einer Funktionalität von 2 bis 3, erhältlich durch bekannte Umsetzung von Polycarbonsäuren mit mehrfunktionellen Alkoholen, und/oder Polycarbonatdiole mit einem Molekulargewicht von 500 bis 1000 eingesetzt. Die Herstellung der Prepolymere und Quasiprepolymere kann in Anwesenheit oder Abwesenheit von üblichen und bekannten, auch unter Komponente (c) beschriebenen Urethanisierungskatalysatoren durchgeführt werden.

In einer bevorzugten Ausführungsform des erfindungsgemäßen Verfahrens werden als Komponente (a) TDI, MDI und/oder Roh-MDI eingesetzt.

Als gegenüber Isocyanaten reaktive Wasserstoffatome aufweisende Verbindungen (b) werden vorzugsweise Polyesterole und besonders bevorzugt Polyetherole mit einer Funktionalität von 2 bis 8, insbesondere von 2 bis 4 und vorzugsweise 2 bis 3, und einem Molekulargewicht im Bereich von 400 bis 8500, vorzugsweise 1000 bis 6000, eingesetzt. Die Polyetherole können nach bekannten Verfahren, zumeist durch katalytische Anlagerung von Alkylenoxiden, insbesondere Ethylenoxid und/oder Propylenoxid, an H-funktionelle Startsubstanzen, oder durch Kondensation von Tetrahydrofuran, hergestellt werden. Als H-funktionelle Startsubstanzen kommen insbesondere mehrfunktionelle Alkohole und/oder Amine zum Einsatz. Bevorzugt eingesetzte Alkohole sind zweiwertige Alkohole, beispielsweise Ethylenglykol, Propylenglykol oder Butandiole, dreiwertige Alkohole, beispielsweise Glyzerin, Trimethylolpropan oder Pentaerythrit, sowie höherwertige Alkohole, wie Zuckeralkohole, beispielsweise Sucrose, Glucose oder Sorbit. Bevorzugt eingesetzte Amine sind aliphatische Amine mit bis zu 10 Kohlenstoffatomen, beispielsweise Ethylendiamin, Diethylentriamin, Propylendiamin, aromatische Amine, beispielsweise Toluylendiamin oder Diaminodiphenylmethan, sowie Aminoalkohole, wie Ethanolamin oder Diethanolamin. Als Polyetheralkohole können auch polymermodifizierte Polyetheralkohole eingesetzt werden. Diese werden zumeist durch in-situ-Polymerisation von olefinisch ungesättigten Monomeren, insbesondere Acrylnitril und/oder Styrol in den Polyetheralkoholen hergestellt. Zu den polymermodifizierten Polyetheralkoholen gehören auch Polyharnstoffdispersionen enthaltende Polyetheralkohole.

Zu den Verbindungen mit mindestens zwei aktiven Wasserstoffatomen gehören auch die Kettenverlängerungs- und Vernetzungsmittel, die gegebenenfalls mitverwendet werden können. Als Kettenverlängerungs- und Vernetzungsmittel werden vorzugsweise 2- und 3-funktionelle Alkohole mit Molekulargewichten unter 400, insbesondere im Bereich von 60 bis 150 eingesetzt. Beispiele sind Ethylenglykol, Propylenglykol, Diethylenglykol und Butandiol-1,4. Als Vernetzungsmittel können auch Diamine eingesetzt werden. Falls Kettenverlängerungs- und Vernetzungsmittel eingesetzt werden, beträgt deren Menge vorzugsweise bis zu 5 Gew.-%, bezogen auf das Gewicht der Polyolkomponente.

Der Reaktionsmischung zur Herstellung der erfindungsgemäßen Polyurethanschaumstoffe werden weitere Hilfsmittel und/oder Zusatzstoffe (c) zugegeben. Genannt seien beispielsweise Katalysatoren, Treibmittel, Schaumstabilisatoren, Flammschutzmittel, Füllstoffe, Farbstoffe, Pigmente und Hydrolyseschutzmittel sowie fungistatische und bakteriostatisch wirkende Substanzen.

Als Katalysatoren für die Herstellung der erfindungsgemäßen Polyurethanschaumstoffe werden die üblichen und bekannten Polyurethanbildungskatalysatoren eingesetzt, beispielsweise organische Zinnverbindungen, wie Zinndiacetat, Zinndioctoat, Dialkylzinndilaurat, und/oder stark basische Amine, wie Triethylamin, Pentamethyldiethylentriamin, Tetramethyldiaminoethylether, 1,2-Dimethylimidazol oder vorzugsweise Triethylendiamin. Die Katalysatoren werden vorzugsweise in einer Menge von 0,01 bis 5 Gew.-%, insbesondere 0,05 bis 2 Gew.-%, bezogen auf das Gewicht der Polyolkomponente, eingesetzt.

Als Treibmittel zur Herstellung der Polyurethanschaumstoffe wird bevorzugt Wasser verwendet, das mit den Isocyanatgruppen unter Freisetzung von Kohlendioxid reagiert. Gemeinsam mit oder an Stelle von Wasser können auch physikalisch wirkende Treibmittel, beispielsweise Kohlenwasserstoffe, wie n-, iso- oder Cyclopentan, oder halogenierte Kohlenwasserstoffe, wie Tetrafluorethan, Pentafluorpropan, Heptafluorpropan, Pentafluorbutan, Hexafluorbutan oder Dichlormonofluorethan, eingesetzt werden. Die Menge des physikalischen Treibmittels liegt dabei vorzugsweise im Bereich zwischen 1 bis 15 Gew.-%, insbesondere 1 bis 10 Gew.-%, die Menge an Wasser vorzugsweise im Bereich zwischen 0,5 bis 10 Gew.-%, insbesondere 1 bis 5 Gew.-%, jeweils bezogen auf das Gewicht der Polyolkomponente.

Durch eine geeignete Auswahl von Schaumstabilisatoren, vorzugsweise eingesetzt werden Silikonstabilisatören, lässt sich die Offenzelligkeit der Polyurethanschaumstoffe beeinflussen. Eine Verringerung des Stabilisatoranteils bewirkt dabei häufig eine Porenvergrößerung.

Weitere Angaben über die oben genannten und weitere verwendbare Ausgangsstoffe finden sich beispielsweise im oben zitierten Kunststoff-Handbuch, Band VII, Polyurethane.

Bei der Herstellung der erfindungsgemäßen Polyurethane werden die Polyisocyanate und die Verbindungen mit mindestens zwei mit Isocyanatgruppen reaktiven Wasserstoffatomen in einer solchen Menge zusammengebracht, dass das Äquivalenzverhältnis von Isocyanatgruppen zur Summe der aktiven Wasserstoffatome 0,8 :1 bis 3,0:1, vorzugsweise 1,2:1 bis 2,0:1 beträgt, oder mit einem Isocyanatüberschuss wie weiter oben beschrieben verarbeitet.

Die Herstellung der Polyurethanschaumstoffe erfolgt vorzugsweise nach dem one-shot-Verfahren, beispielsweise mit Hilfe der Hochdruck- oder Niederdrucktechnik.

Besonders vorteilhaft ist es, nach dem üblichen sogenannten Zweikomponentenverfahren zu arbeiten, bei dem eine Polyol- und eine Isocyanatkomponente hergestellt und verschäumt werden. Die Komponenten werden vorzugsweise bei einer Temperatur im Bereich zwischen 15 bis 90°C, vorzugsweise 20 bis 60°C und besonders bevorzugt 20 bis 35°C zur Reaktion gebracht.

Die nach dem erfindungsgemäßen Verfahren hergestellten Polyurethanschaumstoffe mit reaktiven Gruppen an der Schaumstoffoberfläche weisen vorzugsweise eine Dichte von 10 bis 800 kg/m³, besonders bevorzugt von 30 bis 100 kg/m³ und insbesondere von 30 bis 80 kg/m³, auf.

Zur Verbesserung der Kontaktfläche mit dem jeweiligen Medium können die geschäumten Körper in geometrische Formen gebracht werden (Würfel, Kugeln usw.). Möglich und sinnvoll sind auch Auskleidungen von Reaktionsgefäßen, Rohren usw. Durch eine entsprechende Variation der Zellgröße kann der erforderliche Pumpendruck bei Anwendung kompakter Materialien optimiert werden.

Gut geeignet ist die Verwendung grobkörniger Partikel, die beispielsweise durch Raspeln oder Mahlen der Polyurethanschaumstoffe hergestellt werden können. Diese Materialien können z.B. in ein Reaktorrohr eingebracht werden.

Die vorliegende Erfindung soll anhand der nachstehenden Beispiele näher erläutert werden.

Als Testreaktion zur Überprüfung der Wirksamkeit der Lipase diente die enzymkatalysierte Veresterung von 1-Phenylethanol mit Vinylpropionat.

Dabei wurde folgendermaßen verfahren: Die immobilisierte Lipase wurde mit einer Substratlösung (siehe Modellreaktion) versetzt und bei Raumtemperatur geschüttelt. Der Umsatz wurde mittels Gaschromatographie verfolgt. Der Maximalumsatz betrug 50 Gew.-%. Nach 24 Stunden wurde filtriert und die zurückgewonnene Lipase mit neuem Substrat versetzt und erneut geschüttelt. 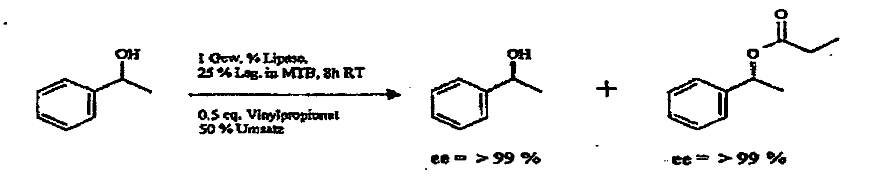
- MTB: Methyltertiärbutylether
- ee: Enantiomerenüberschuss

Die eingesetzten Schaumrezepturen sind in der folgenden Tabelle zusammengefaßt:

| | System 1 | System 2 |
|---|---|---|
| Polyol 1 | 14 | |
| Polyol 2 | 53 | |
| Polyol 3 | 4,3 | |
| Polyol 4 | | 68,8 |
| Polyol 5 | 5 | |
| Polyol 6 | | 23,9 |
| Polyol 7 | 13 | |
| Polyol 8 | 5 | |
| Glycerin | | 1,4 |
| Lupragen® N600 | 0,3 | |
| DMCHA | | 2,5 |
| TMR-2 | 0,1 | |
| VP 9104 | 1 | |
| B 8409 | | 1 |
| B 1903 | 0, 7 | |
| B 8919 | 1 | |
| Wasser | 2,6 | 2,4 |

Als Isocyanatkomponente diente in allen Fällen Lupranat® M20A (BASF).

| | |
|---|---|
| Polyol 1 - | OH-Zahl 400 mg KOH/g, Polyetheralkohol auf Basis von Propylenoxid, Starter Glycerin; |
| Polyol 2 - | OH-Zahl 240 mg KOH/g, Polyesteralkohol auf Basis von Phthalsäureanhydrid; |
| Polyol 3 - | OH-Zahl 400 mg KOH/g, Polyetheralkohol auf Basis von Propylenoxid, Starter Saccharose; |
| Polyol 4 - | OH-Zahl 490 mg KOH/g, Polyetheralkohol auf Basis von Propylenoxid, Starter Saccharose; |
| Polyol 5 - | OH-Zahl 55 mg KOH/g, Polyetheralkohol auf Basis von Propylenoxid, Starter Propylenglykol; |
| Polyol 6 - | OH-Zahl 105 mg KOH/g, Polyetheralkohol auf Basis von Propylenoxid, Starter Propylenglykol; |
| Polyol 7 - | OH-Zahl 250 mg KOH/g, Polyetheralkohol auf Basis von Propylenoxid, Starter Propylenglykol; |
| Polyol 8 - | OH-Zahl 385 mg KOH/g, Polyesteralkohol auf Basis von Adipinsäure/Phthalsäureanhydrid; |
| Lupragen® N 600 - | Aminkatalysatoren (BASF); |
| VP 9104 - | Katalysator (BASF); |
| DMCHA - | Dimethylcyclohexylamin (Katalysator); |
| TMR-2 - | Aminkatalysator (Air Products); |
| B 1903, B 8419, B 8409 - | Sililkonstabilisatoren (Goldschmidt); |
| Lupranat® M20A - | NCO 31,6 % Polyphenylenpolymethylenpolyisocyanat; |

### Beispiel 1 a (Vergleich)

Formulierung 1 wurde bei einer Kennzahl von 180 zu einem Schaumstoff umgesetzt. Dieser Schaumstoff wurde in der Umgebungsluft drei Wochen gelagert und danach mit einer Raspel zerkleinert. IR-spektroskopisch konnte eine deutliche Abnahme der reaktiven NCO-Gruppen gefunden werden.

Die gefriergetrocknete Lipase wurde in einer Suspension in Methyltertiärbutylether mit dem geraspelten Schaumstoff in Kontakt gebracht und ca. 15 Minuten gerührt. Danach wurde im Rotationsverdampfer das Lösungsmittel entfernt. Es wurde eine Enzymbeladung von 5 Gew.-% eingestellt.

Der so hergestellte Enzym-Träger-Verbund wurde hinsichtlich seiner Aktivität bei der chiralen Umesterung (siehe Testreaktion) eingesetzt. Nach 2 Durchläufen kam es zu einem signifikanten Aktivitätsabfall.

### Beispiel 1 b

Formulierung 1 wurde bei einer Kennzahl von 180 zu einem Schaumstoff umgesetzt. Dieser Schaumstoff wurde unmittelbar nach der Herstellung (ohne Lagerung) mit einer Raspel zerkleinert.

Die gefriergetrocknete Lipase wurde in einer Suspension in Methyltertiärbutylether mit dem geraspelten Schaumstoff in Kontakt gebracht und ca. 15 Minuten gerührt. Danach wurde im Rotationsverdampfer das Lösungsmittel entfernt. Es wurde eine Enzymbeladung von 5 Gew.-% eingestellt.

Der so hergestellte Enzym-Träger-Verbund wurde hinsichtlich seiner Aktivität bei der chiralen Umesterung (siehe Testreaktion) eingesetzt. Nach 20 Durchläufen kam es zu einem signifikanten Aktivitätsabfall.

### Beispiel 2

Formulierung 1 wurde bei einer Kennzahl von 110 zu einem Schaumstoff umgesetzt. Dieser Schaumstoff wurde zwei Wochen gelagert und danach mit einer Raspel zerkleinert. Vor der Fixierung der Lipase wurde der geraspelte Schaumstoff für 1 Stunde bei 80°C ausgeheizt und dann mit einer Lösung von Lupranat MI in Methyltertiärbutylether in Kontakt gebracht (0,5 Gew.-% NCO-Gruppen zusätzlich auf der Polyurethanoberfläche). Das Lösungsmittel wurde nach einer Rührzeit von ca. 15 Minuten vom Polyurethanträger entfernt.

Daraufhin wurde die gefriergetrocknete Lipase in einer Suspension in Methyltertiärbutylether mit dem geraspelten Schaumstoff in Kontakt gebracht und ca. 15 Minuten gerührt. Danach wurde im Rotationsverdampfer das Lösungsmittel entfernt. Es wurde eine Enzymbeladung von 5 Gew.-% eingestellt.

Der so hergestellte Enzym-Träger-Verbund wurde hinsichtlich seiner Aktivität bei der chiralen Umesterung (siehe Testreaktion) eingesetzt. Nach 14 Durchläufen kam es zu einem signifikanten Aktivitätsabfall.

### Beispiel 3

Formulierung 1 wurde bei einer Kennzahl von 180 zu einem Schaumstoff umgesetzt. Unmittelbar nach der Verschäumung wurde das Polyurethan mit einer Raspel zerkleinert. Vor der Fixierung der Lipase wurde der geraspelte Schaumstoff für 1 Stunde bei 80°C ausgeheizt.

Danach wurde eine überstehende Fermenterbrühe mit dem geraspelten Schaumstoff in Kontakt gebracht und ca. 15 Minuten gerührt. Nachfolgend wurde im Rotationsverdampfer das überschüssige Wasser schonend entfernt. Es wurde eine Enzymbeladung von 25000 U/g eingestellt.

Der so hergestellte Enzym-Träger-Verbund wurde hinsichtlich seiner Aktivität bei der chiralen Umesterung (siehe Testreaktion) eingesetzt. Nach 29 Durchläufen kam es zu einem signifikanten Aktivitätsabfall.

### Beispiel 4

Formulierung 5 wurde bei einer Kennzahl von 110 zu einem Schaumstoff umgesetzt. Zusätzlich war der polyolischen Komponente 5 Gew.-% Glycidol zugesetzt worden. Dieser Schaumstoff wurde mit einer Raspel zerkleinert.

Daraufhin wurde die gefriergetrocknete Lipase in einer Suspension in Methyltertiärbutylether mit dem geraspelten Schaumstoff in Kontakt gebracht und ca. 15 Minuten gerührt. Danach wurde im Rotationsverdampfer das Lösungsmittel entfernt. Es wurde eine Enzymbeladung von 5 Gew.-% eingestellt.

Der so hergestellte Enzym-Träger-Verbund wurde hinsichtlich seiner Aktivität bei der chiralen Umesterung (siehe Testreaktion) eingesetzt. Nach 6 Durchläufen kam es zu einem signifikanten Aktivitätsabfall.

### Beispiel 5

Formulierung 5 wurde bei einer Kennzahl von 180 zu einem Schaumstoff umgesetzt. Dieser Schaumstoff wurde zwei Wochen gelagert und danach mit einer Raspel zerkleinert. Vor der Fixierung der Lipase wurde der geraspelte Schaumstoff mit einer Lösung von Lupranat MI in Methyltertiärbutylether in Kontakt gebracht (1 Gew.-% NCO-Gruppen zusätzlich auf der Polyurethanoberfläche). Das Lösungsmittel wurde nach einer Rührzeit von ca. 15 Minuten vom Polyurethanträger entfernt. In einem vorgelagerten Schritt waren 5 Gew.-% eines C₉-Calixarenderivates analog zur NCO-Gruppenaufträgerung auf die Schaumstoffoberfläche aufgebracht worden.

Daraufhin wurde die gefriergetrocknete Lipase in einer Suspension in Methyltertiärbutylether mit dem geraspelten Schaumstoff in Kontakt gebracht und ca. 15 Minuten gerührt. Danach wurde im Rotationsverdampfer das Lösungsmittel entfernt. Es wurde eine Enzymbeladung von 5 Gew.-% eingestellt.

Der so hergestellte Enzym-Träger-Verbund wurde hinsichtlich seiner Aktivität bei der chiralen Umesterung (siehe Testreaktion) eingesetzt. Nach 10 Durchläufen kam es zu einem signifikanten Aktivitätsabfall.

## Patentansprüche

1. Verfahren zur Herstellung von kovalent gebundenen biologisch aktiven Stoffen an Polyurethanschaumstoffen, **dadurch gekennzeichnet, dass** an der Schaumstoffoberfläche Isocyanatgruppen sowie gegebenenfalls weitere reaktive Gruppen erzeugt und an diesen biologisch aktive Stoffe kovalent fixiert werden.

2. Verfahren nach Anspruch 1, **dadurch gekennzeichnet, dass** durch Herstellung des Polyurethanschaumstoffes mit einem Isocyanatüberschuss zugängliche reaktive NCO-Gruppen an der Schaumstoffoberfläche erzeugt werden.

3. Verfahren nach Anspruch 1 oder 2, **dadurch gekennzeichnet, dass** durch eine nachträgliche Tränkung des Polyurethanschaumstoffes mit Di- und/oder Polyisocyanaten reaktive NCO-Gruppen an der Schaumstoffoberfläche erzeugt werden.

4. Verfahren nach einem der Ansprüche 1 bis 3, **dadurch gekennzeichnet, dass** durch Einsatz zellöffnender Materialien und/oder Zerkleinerung des Schaumstoffes die Anzahl zugänglicher reaktiver NCO-Gruppen an der Schaumstoffoberfläche erhöht wird.

5. Verfahren nach einem der Ansprüche 1 bis 4, **dadurch gekennzeichnet, dass** durch Verwendung von OH-funktionellen Epoxiden bei der Herstellung der Polyurethanschaumstoffe reaktive Epoxidgruppen an der Schaumstoffoberfläche erzeugt werden.

6. Verfahren nach einem der Ansprüche 1 bis 5, **dadurch gekennzeichnet, dass** durch Verwendung von säuregruppentragenden Verbindungen bei der Herstellung der Polyurethanschaumstoffe reaktive Säuregruppen an der Schaumstoffoberfläche erzeugt werden.

7. Verfahren nach einem der Ansprüche 1 bis 6, **dadurch gekennzeichnet, dass** durch Verwendung von Phenolen bei der Herstellung der Polyurethanschaumstoffe OH-Gruppen an der Schaumstoffoberfläche erzeugt werden.

8. Verfahren nach einem der Ansprüche 1 bis 7, **dadurch gekennzeichnet, dass** die biologisch aktiven Materialien mit dem Polyurethanschaum in Kontakt gebracht werden.

9. Verfahren nach einem der Ansprüche 1 bis 8, **dadurch gekennzeichnet, dass** die biologisch aktiven Materialien Enzyme sind.

10. Mit biologisch aktiven Materialien geträgerte Polyurethanschaumstoffe, herstellbar nach einem der Ansprüche 1 bis 9.

11. Polyurethanschaumstoffe, gemäß Anspruch 10, **dadurch gekennzeichnet, dass** die Quellung der geträgerten biologisch aktiven Materialien weniger als 10% beträgt.

12. Verwendung der mit biologisch aktiven Materialien geträgerten Polyurethanschaumstoffe gemäß Anspruch 10 für chirale Synthesen.

13. Verwendung der mit biologisch aktiven Materialien geträgerten Polyurethanschaumstoffe gemäß Anspruch 10 für selektive Umesterungsreaktionen.

## Claims

1. A process for the preparation of covalently bonded biologically active substances on polyurethane foams, wherein isocyanate groups and, if required, further reactive groups are produced on the foam surface and biologically active substances are covalently immobilized on said groups.

2. A process as claimed in claim 1, wherein, by preparing the polyurethane foam using an isocyanate excess, accessible reactive NCO groups are produced on the foam surface.

3. A process as claimed in claim 1 or 2, wherein, by subsequently impregnating the polyurethane foam with di- and/or polyisocyanates, reactive NCO groups are produced on the foam surface.

4. A process as claimed in any of claims 1 to 3, wherein, by using cell-opening materials and/or comminuting the foam, the number of accessible reactive NCO groups on the foam surface is increased.

5. A process as claimed in any of claims 1 to 4, wherein, by using OH-functional epoxides in the preparation of the polyurethane foams, reactive epoxide groups are produced on the foam surface.

6. A process as claimed in any of claims 1 to 5, wherein, by using compounds carrying acid groups in the preparation of the polyurethane foams, reactive acid groups are produced on the foam surface.

7. A process as claimed in any of claims 1 to 6, wherein, by using phenols in the preparation of the polyurethane foams, OH groups are produced on the foam surface.

8. A process as claimed in any of claims 1 to 7, wherein the biologically active materials are brought into contact with the polyurethane foam.

9. A process as claimed in any of claims 1 to 8, wherein the biologically active materials are enzymes.

10. A polyurethane foam supporting biologically active materials, which is preparable as claimed in any of claims 1 to 9.

11. A polyurethane foam as claimed in claim 10, wherein the swelling of the supported biologically active materials is less than 10%.

12. The use of a polyurethane foam supporting biologically active materials, as claimed in claim 10, for chiral syntheses.

13. The use of a polyurethane foam supporting biologically active materials, as claimed in claim 10, for selective transesterification reactions.

## Revendications

1. Procédé pour la préparation de substances à activité biologique liées par covalence à des matières alvéolaires de polyuréthane, **caractérisé par le fait qu'**on produit sur la surface de la matière alvéolaire des groupes isocyanate, ainsi qu'éventuellement d'autres groupes réactifs et on fixe par covalence sur ces groupes des substances à activité biologique.

2. Procédé selon la revendication 1, **caractérisé par le fait que** par la préparation de la matière alvéolaire de polyuréthane avec un excès d'isocyanate sont produits des groupes NCO réactifs accessibles sur la surface de la matière alvéolaire.

3. Procédé selon la revendication 1 ou 2, **caractérisé par le fait que** par une imprégnation complémentaire de la matière alvéolaire de polyuréthane avec des di- et/ou polyisocyanates sont produits des groupes NCO réactifs sur la matière alvéolaire.

4. Procédé selon l'une des revendications 1 à 3, **caractérisé par le fait que** par utilisation de matières pour l'ouverture de cellules et/ou la fragmentation de la matière alvéolaire on augmente le nombre des groupes NCO réactifs accessibles sur la surface de la matière alvéolaire.

5. Procédé selon l'une des revendications 1 à 4, **caractérisé par le fait que** par l'utilisation d'époxydes OH-fonctionnels lors de la préparation des matières alvéolaires de polyuréthane on produit des groupes époxyde réactifs sur la surface de la matière alvéolaire.

6. Procédé selon l'une des revendications 1 à 5, **caractérisé par le fait que** par l'utilisation de composés portant des groupes acide lors de la préparation des matières alvéolaires de polyuréthane on produit des groupes acide réactifs sur la surface de la matière alvéolaire.

7. Procédé selon l'une des revendications 1 à 6, **caractérisé par le fait que** par l'utilisation de phénols lors de la préparation des matières alvéolaires de polyuréthane on produit des groupes OH sur la surface de la matière alvéolaire.

8. Procédé selon l'une des revendications 1 à 7, **caractérisé par le fait que** les matières biologiquement actives sont mises en contact avec la matière alvéolaire de polyuréthane.

9. Procédé selon l'une des revendications 1 à 8, **caractérisé par le fait que** les matières biologiquement actives sont des enzymes.

10. Matières alvéolaires de polyuréthane portant des matières biologiquement actives, pouvant être préparées selon l'une des revendications 1 à 9.

11. Matières alvéolaires de polyuréthane selon la revendication 10, **caractérisées par le fait que** le gonflement des matières biologiquement actives sur support est inférieur à 10%.

12. Utilisation des matières alvéolaires en polyuréthane portant des matières biologiquement actives selon la revendication 10 pour des synthèses chirales.

13. Utilisation des matières alvéolaires en polyuréthane portant des matières biologiquement actives selon la revendication 10 pour des réactions de trans-estérification sélective.
